(19) Europäisches Patentamt / European Patent Office / Office européen des brevets

(11) **EP 2 704 629 B1**

(12) **EUROPÄISCHE PATENTSCHRIFT**

(45) Veröffentlichungstag und Bekanntmachung des Hinweises auf die Patenterteilung:
**28.12.2016 Patentblatt 2016/52**

(21) Anmeldenummer: **12718076.8**

(22) Anmeldetag: **26.04.2012**

(51) Int Cl.:
*A61B 5/0476* *(2006.01)*      *A61B 5/04* *(2006.01)*
*A61B 5/00* *(2006.01)*      *A61B 5/0478* *(2006.01)*

(86) Internationale Anmeldenummer:
**PCT/EP2012/001794**

(87) Internationale Veröffentlichungsnummer:
**WO 2012/150015 (08.11.2012 Gazette 2012/45)**

(54) **VERFAHREN ZUR BEWUSSTSEINS- SOWIE SCHMERZÜBERWACHUNG, MODUL ZUR ANALYSE VON EEG-SIGNALEN UND EEG-NARKOSEMONITOR**

METHOD FOR CONSCIOUSNESS AND PAIN MONITORING, MODULE FOR ANALYZING EEG SIGNALS AND EEG NARCOSIS MONITOR

PROCÉDÉ POUR LA SURVEILLANCE DE LA CONSCIENCE ET DE LA DOULEUR, MODULE D'ANALYSE DE SIGNAUX EEG ET MONITEUR DE NARCOSE PAR EEG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.05.2011 DE 102011100173**
**06.10.2011 DE 102011115116**

(43) Veröffentlichungstag der Anmeldung:
**12.03.2014 Patentblatt 2014/11**

(73) Patentinhaber:
• **Jordan, Denis**
  **81669 München (DE)**
• **Kochs, Eberhard**
  **81927 München (DE)**

(72) Erfinder:
• **Jordan, Denis**
  **81669 München (DE)**
• **Kochs, Eberhard**
  **81927 München (DE)**

(74) Vertreter: **Prinz & Partner mbB**
**Patent- und Rechtsanwälte**
**Rundfunkplatz 2**
**80335 München (DE)**

(56) Entgegenhaltungen:
**WO-A1-2009/079384**

• **Staniek, Matthäus: "Symbolische Transferentropie: Charakterisierung gerichteter Interaktionen in nichtlinearen dynamischen Netzwerken", 14. April 2010 (2010-04-14), http://hss.ulb.uni-bonn.de/2010/2369/2369. pdf, XP009161397, Seiten 13-24**
• **VAKORIN V A ET AL: "Exploring transient transfer entropy based on a group-wise ICA decomposition of EEG data", NEUROIMAGE, ACADEMIC PRESS, ORLANDO, FL, US, Bd. 49, Nr. 2, 15. Januar 2010 (2010-01-15), Seiten 1593-1600, XP026796284, ISSN: 1053-8119 [gefunden am 2009-08-18]**

**Beschreibung**

Die Erfindung betrifft ein Verfahren zur EEG basierten Bewusstseins- sowie Schmerzüberwachung, vorzugsweise zur Narkoseüberwachung, ein Modul zur Analyse von EEG-Signalen, und einen EEG-Narkosemonitor.

Heutzutage wird Allgemeinanästhesie durch eine Kombination verschiedener Anästhetika herbeigeführt und in erster Linie anhand unspezifischer Überwachungsparameter (z.B. Blutdruck, Herzfrequenz, Schwitzen) überwacht. Diese surrogaten Standardparameter reflektieren zwar Effekte der zentralen Anästhetikawirkung, ermöglichen jedoch keinen direkten Aufschluss über Vorgänge im Gehirn, dem Hauptwirkort der hypnotischen Komponente der Anästhesie. Unter diesen Voraussetzungen besteht ein Restrisiko für intraoperative Wachheit, die für den Patienten zu einer bewussten Erinnerung von Ereignissen während eines Eingriffs und einer gravierenden postoperativen neurokognitiven Belastungsstörung führen kann.

Konventionelle Überwachungsmonitore, im Folgenden auch Standardüberwachungsmonitore genannt, ermöglichen die Messung und Überwachung von Herzrhythmus, Blutdruck und anderen nicht EEG-basierten Vitalparametern, im Folgenden auch Standardparameter genannt, eines Patienten.

Um oben genanntes Restrisiko zu verringern, lässt sich das Gehirn mithilfe des spontanen Elektroenzephalogramms (EEG) spezifischer überwachen als mit den Standardparametern. Aus dem komplexen EEG-Signal werden EEG-Parameter berechnet, durch welche eine Quantifizierung der hypnotischen Komponente der Anästhesie ("Narkosetiefe"), insbesondere eine sichere Unterscheidung von Wachheit und Bewusstlosigkeit, erreicht werden soll. Als Analysemethoden werden unterschiedliche mathematische Verfahren verwendet, beispielsweise die klassische lineare Spektralanalyse. Da das EEG von einem nichtlinearen dynamischen System erzeugt wird, können spezifische Charakteristika der EEG-Signale außerhalb des Amplitudenspektrums liegen.

Aus WO 2009/079384 A1 ist ein Verfahren bekannt, mit dem EEG-Signale mittels Transferentropie ausgewertet werden können. Des Weiteren ist ein EEG-Monitor gezeigt, der die EEG-Signale darstellen kann.

Aus der Dissertation "Symbolische Transferentropie: Charakterisierung gerichteter Interaktionen in nichtlinearen dynamischen Netzwerken", 14. April 2010 von Matthäus Staniek ist es bekannt, EEG-Signale mittels symbolischer Transferentropie auszuwerten.

Aufgabe der Erfindung ist es, ein optimiertes Verfahren zur Bewusstseins-("Narkosetiefe", hypnotische Komponente der Anästhesie, intraoperative Wachheit, Sedierung, Koma) und/oder Schmerzüberwachung (Analgesie) durch eine verbesserte Auswertung der nichtlinearen dynamischen Eigenschaften des EEG-Signals bereitzustellen, sowie ein Modul zur Analyse von EEG-Signalen und einen EEG-Narkosemonitor, welche die Durchführung eines entsprechend verbesserten Verfahrens ermöglichen.

Diese Aufgabe wird durch ein Verfahren zur Bewusstseins- sowie Schmerzüberwachung, vorzugsweise zur Narkoseüberwachung, gelöst, bei dem EEG-Signale mittels symbolischer Transferentropie ausgewertet werden, zur Unterscheidung zwischen Wachheit und Bewusstlosigkeit, wobei ein Indikatorwert zur Unterscheidung zwischen Wachheit und Bewusstlosigkeit basierend auf der Auswertung der EEG-Signale mittels symbolischer Transferentropie ermittelt wird. Die symbolische Transferentropie ermöglicht eine Quantifizierung des Informationsflusses zwischen zwei dynamischen Systemen (im Folgenden System X und System Y). Weil angenommen wird, dass abbrechende kortikale Integration bei Bewusstseinsverlust mit einer Änderung des Informationstransfers auf elektrophysiologischer Ebene zusammenhängt, lässt sich auf diese Weise beispielsweise die hypnotische Komponente bei der Narkoseüberwachung beurteilen. Die symbolische Transferentropie ist in der Lage, spezifisch diesen mechanistischen Vorgang des Bewusstseinsverlustes zu quantifizieren. Dabei werden anstelle von Signalamplituden nur deren Rangordnungen analysiert und auf diese Weise eine robuste Analyse erreicht (geringe Empfindlichkeit gegenüber Rauschen und Signalstörungen). Durch den ordinalen Ansatz lässt sich zudem das EEG mit Hilfe einer geringen Anzahl von Datenpunkten analysieren. Aufgrund des Indikatorwerts kann eine einfache und schnelle Bewertung der Ergebnisse der Analyse der EEG-Signale, beispielsweise durch einen Arzt bei einer Narkoseüberwachung, ermöglicht werden.

Der Indikatorwert des Bewusstseins- und/oder Schmerzzustandes wird vor allem zur Unterscheidung zwischen Wachheit und Bewusstlosigkeit basierend auf der Auswertung der EEG-Signale mittels symbolischer Transferentropie und möglicher weiterer Parameter des EEG und/oder der Standardüberwachung (Herzkreislaufsystem, Atmung sowie Patientendaten und Medikamenteninformationen) ermittelt. Die Kombination der symbolischen Transferentropie mit weiteren Parametern zu einem Indikator kann mit Hilfe einer Fuzzy Logik, neuronalen Netzen, Support Vector Machines oder Regressionen erfolgen. Der Indikator kann dazu verwendet werden, die hypnotische und/oder analgetische Komponente der Anästhesie zu überwachen oder automatisch zu steuern.

Eine hohe Zuverlässigkeit des Verfahrens kann erreicht werden, indem die EEG-Signale von Elektroden, vorzugsweise Elektrodenpaare, abgeleitet werden, die an besonders geeigneten Positionen platziert sind. Dabei können auch mehrere Elektrodenpaare ausgewertet werden.

Es können insbesondere intrafrontale, frontal-parietale, frontal-temporale, bitemporale oder frontal-okzipitale Elektrodenableitungen verwendet werden.

Um den Einfluss des auszuwertenden EEG-Signale von unerwünschten überlagerten Muskelaktivitätssignalen (Elektromyografie; EMG) zu reduzieren, das heißt um das Signal-Rauschverhältnis (SNR) der gemessenen EEG-Signale zu erhöhen, werden die EEG-Signale vorzugsweise vor einer Berechnung der symbolischen Transferentropie mit einer oberen Grenzfrequenz von höchstens 30 Hz tiefpass- oder bandpassgefiltert. Im Falle einer Bandpassfilterung eignen sich Frequenzen innerhalb des EEG $\alpha$- (8-13Hz) und/oder $\beta$-Bandes (13-30Hz) besonders.

Zur Vermeidung von Aliaseffekten bei der Abtastung der EEG-Signale ist eine Abtastfrequenz der EEG-Signale vorgesehen, welche zumindest das Zweifache, vorzugsweise zumindest das Fünffache, der oberen Filterfrequenz beträgt.

Die durch die symbolische Transferentropie analysierten EEG-Signale können zeitliche Messwertsequenzen von 2 bis 30 Sekunden Dauer sein. Somit können auch relativ kurze zeitliche Messwertsequenzen mit dem Verfahren ausgewertet und für die Bestimmung des Bewusstseinszustandes kurze Zeitverzögerungen im Sekundenbereich erreicht werden.

Beispielsweise werden innerhalb zeitlicher Messwertsequenzen x (Messung des Systems X), und y (Messung des Systems Y) aus je N Messwerten der EEG-Signale Teilsequenzen x(i), y(i) der Länge m entlang von x, y gebildet. Ein Lag-Parameter $\tau \geq 1$ bei der Bildung der Teilsequenzen kann zur besseren Entfaltung der aus x(i) und y(i) erzeugten Trajektorien und zu einer genaueren Analyse beitragen. Dabei werden bei der Bildung von x(i) und y(i) nur Amplitudenwerte mit zeitlichem Abstand $\tau/f_s$ verwendet ($f_s$ Abtastfrequenz der Signale x, y).

Symbolische Sequenzen $\hat{x}(i)$, $\hat{y}(i)$ werden daraufhin durch eine Symbolisierung der Teilsequenzen x(i), y(i), vorzugsweise durch Bestimmung der Rangordnung der Amplituden (Ordinalanalyse), erhalten.

Es ist möglich, dass eine direktionale symbolische Transferentropie nach der Formel

$$\text{STEn}_{Y \to X} = \sum_i p(\hat{x}(i+\delta), \hat{x}_k(i), \hat{y}_l(i)) \cdot \log\left( \frac{p(\hat{x}(i+\delta)|\hat{x}_k(i), \hat{y}_l(i))}{p(\hat{x}(i+\delta)|\hat{x}_k(i))} \right)$$

berechnet wird, wobei $\hat{x}_k(i)$ und $\hat{y}_l(i)$ jeweils den k bzw. l letzten symbolischen Sequenzen gemäß der Formeln $\hat{x}_k(i) = \hat{x}(i), \hat{x}(i-1),..., \hat{x}(i-k); \hat{y}_l(i) = \hat{y}(i), \hat{y}(i-1),..., \hat{y}(i-l)$ entsprechen und die Summe über alle Sequenzen $\hat{x}(i+\delta)$, $\hat{x}_k(i)$, $\hat{y}_l(i)$ gebildet wird. Dadurch wird die Wahrscheinlichkeit der extrinsischen Voraussagbarkeit einer Sequenz $\hat{x}(i+\delta)$ mit $\delta > 0$ aus Informationen in $\hat{y}_l(i)$ ausgedrückt. Die direktionale symbolische Transferentropie ist also ein Maß dafür, in wie weit sich Teilsequenzen aus einer Messwertsequenz von vorhergehenden Teilsequenzen aus der anderen Messwertsequenz erklären lassen. Durch Austausch der entsprechenden Teilsequenzen der Systeme X und Y kann entsprechend die direktionale symbolische Transferentropie $\text{STEn}_{X \to Y}$ berechnet werden.

Der Zeitversatz ö wird vorzugsweise so gewählt, dass der Quotient aus Abtastfrequenz und Zeitversatz im Frequenzbereich des EEG $\alpha$-, $\beta$-Bandes liegt.

Es ist zusätzlich möglich, dass ein Richtungsindex $\text{STEn}_{DI}$ nach der Formel $\text{STEn}_{DI} = \text{STEn}_{X \to Y} - \text{STEn}_{X \to Y}$ berechnet wird. Ein Wert von 0 repräsentiert bei vorhandener Kommunikation ($\text{STEn}_{Y \to X} + \text{STEn}_{X \to Y} > 0$) einen ausgeglichenen bidirektionalen Informationsaustausch zwischen X und Y, für positive Werte ist das System *X* vorwiegend der Generator, für negative Werte ist das System Y vorwiegend der Generator.

Die Aufgabe der Erfindung wird ferner durch ein Modul zur Analyse von EEG-Signalen gelöst, welches einen Dateneingang, der EEG-Signale empfangen kann, eine Rechnereinheit, die die EEG-Signale nach einem Verfahren gemäß einem der vorhergehenden Ansprüche auswerten kann, und eine Ausgabeeinheit umfasst, welche einen Indikatorwert zur Unterscheidung zwischen Wachheit und Bewusstlosigkeit ausgeben kann. Dies ermöglicht einen modularen Aufbau eines Systems zur Analyse von EEG-Signalen, wobei beispielsweise das Modul EEG-Signale von einem separaten EEG-Verstärker empfangen kann und den Indikatorwert an einen konventionellen Überwachungsmonitor ausgibt.

Es ist auch möglich, dass das Modul einen EEG-Verstärker umfasst, welcher im Modul fest integriert ist oder eine separate portable vorzugsweise drahtlose Einheit bildet und EEG-Signale an den Dateneingang des Moduls bereitstellt.

Ferner kann das Modul eine Schnittstelle für einen konventionellen Überwachungsmonitor aufweisen, die nicht-EEG-Parameter empfangen kann und wobei die Rechnereinheit ausgebildet ist, um den Indikatorwert unter Einbezug der nicht-EEG-Parameter bestimmen zu können.

Ein erfindungsgemäßer EEG-Narkosemonitor ist ausgebildet, um EEG-Signale zu messen und mittels symbolischer Transferentropie auszuwerten, vorzugsweise nach einem Verfahren der Ansprüche 1 bis 10, um zwischen Wachheit und Bewusstlosigkeit unterscheiden zu können, wobei der EEG-Narkosemonitor ausgebildet ist, um einen Indikatorwert, zur Unterscheidung zwischen Wachheit und Bewusstlosigkeit, zu ermitteln.

Ein wie oben beschriebenes Modul sowie ein eigenständiger EEG-Narkosemonitor kann neben der Narkoseüberwachung oder -steuerung auch in anderen Anwendungsgebieten verwendet werden, insbesondere bei der Patientenüberwachung auf der Intensivstation, beispielsweise bei Sedierung oder Koma, zur Schlafüberwachung in der Schlafforschung oder zur Vigilanzüberwachung von Verkehrsteilnehmern, beispielsweise Piloten, LKW- oder Busfahrern.

Die Erfindung ist in den Ansprüchen definiert; andere Ausführungsbeispiele sind lediglich exemplarisch.

Weitere Merkmale und Vorteile der Erfindung ergeben sich aus der nachfolgenden Beschreibung und aus den Zeichnungen, auf die Bezug genommen wird. In den Zeichnungen zeigen:

- Figur 1 einen erfindungsgemäßen EEG-Narkosemonitor;
- Figur 2 einen Narkosemonitor mit einem erfindungsgemäßen Modul zur Analyse von EEG-Signalen;
- Figur 3 ein erfindungsgemäßes Modul zur Analyse von EEG-Signalen;
- Figur 4 ein Ablaufdiagramm eines erfindungsgemäßen Verfahrens zur Bewusstseinsüberwachung;
- Figur 5 eine grafische Darstellung der symbolischen Transferentropie von EEG-Signalen (64 Kanäle) bei entspannter Wachheit und Bewusstlosigkeit; und
- Figur 6 ein Ablaufdiagramm der Bestimmung eines Indikatorwerts aus einzelnen EEG- sowie optionalen nicht-EEG-Parametern gemäß einem erfindungsgemäßen Verfahren.

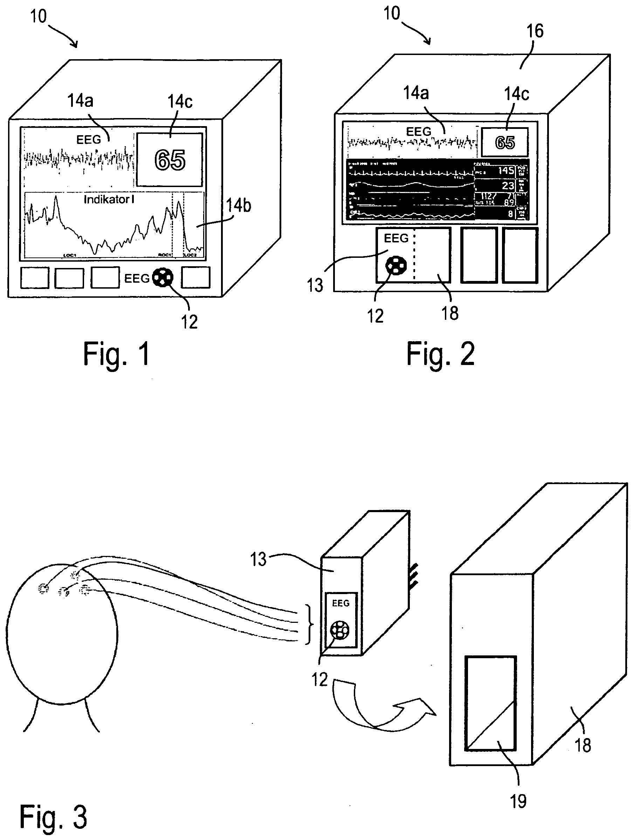

Figur 1 zeigt einen EEG-Narkosemonitor 10, mit dem EEG-Signale mittels symbolischer Transferentropie ausgewertet werden können. Der Narkosemonitor 10 hat einen Anschluss 12 für eine Mehrzahl von EEG-Elektroden, die auf der Kopfhaut eines Patienten angeordnet werden und mit denen das EEG abgeleitet wird. Der Narkosemonitor 10 wertet die von den Elektroden empfangenen EEG-Signale mittels symbolischer Transferentropie aus, wobei ein Indikatorwert I, zur Unterscheidung zwischen Wachheit und Bewusstlosigkeit, ermittelt wird.

Der Narkosemonitor 10 umfasst eine erste Anzeige 14a, welche die EEG-Signale, eine zweite Anzeige 14b, die den zeitlichen Verlauf des Indikatorwerts I, sowie eine dritte Anzeige 14c, welche den momentanen Indikatorwert I anzeigt. Dies ermöglicht es einem Arzt, bei der Narkoseüberwachung eine einfache und schnelle Einschätzung der Narkosetiefe durchzuführen.

Der Narkosemonitor 10 gemäß Figur 1 ist als eigenständiges Gerät ausgebildet, wobei neben der Ermittlung und Anzeige des Indikatorwerts I als Ergebnis der EEG-Signalanalyse mittels symbolischer Transferentropie und möglicher zusätzlicher EEG- und Standardparameter auch weitere Funktionen zur Auswertung der EEG-Signale und/oder zur Bewertung des Bewusstseinszustandes und/oder des Schmerzes vorgesehen sein können.

Figur 2 zeigt eine alternative Ausführungsform eines Narkosemonitors 10 mit einem herkömmlichen, bekannten Standardüberwachungsmonitor der Anästhesie 16, welcher mit einem zusätzlichen Modul 18 ausgestattet ist, der eine Auswertung der EEG-Signale mittels symbolischer Transferentropie ermöglicht.

Das Modul 18 weist neben Anschlüssen für die Stromversorgung durch den Standardüberwachungsmonitor 16 einen Dateneingang mit integriertem EEG-Verstärker 13, welcher EEG-Signale direkt messen oder empfangen kann, eine Rechnereinheit, welche die EEG-Signale mittels der symbolischen Transferentropie und möglicher weiterer EEG- und Standardparameter auswerten kann, und eine Schnittstelle zum Standardüberwachungsmonitor 16, durch welche der berechnete Indikator aus symbolischer Transferentropie mit möglicher Kombination aus weiteren EEG-Parametern mit/ohne Einbezug von Standardparametern und das gemessene EEG auf die Ausgabeeinheit dargestellt wird. In der gezeigten Ausführungsform wird der Indikatorwert I an den Standardüberwachungsmonitor 16 übermittelt und wird auf dessen Anzeige 14c angezeigt.

Figur 3 zeigt eine Variante eines Moduls 18, welches im Gegensatz zum integrierten EEG-Verstärker mit Buchse 12 einen mobilen EEG-Verstärker 13 umfasst. Der EEG-Verstärker 13 ist als separate portable Einheit mit einem Akkumulator zur Energieversorgung ausgebildet und ermöglicht eine drahtlose Übertragung der EEG-Signale an den Dateneingang des Moduls 18. Somit kann der EEG-Verstärker nahe dem Patienten platziert werden, ohne den Standort des Monitors einzuschränken.

Das Modul 18 weist einen Steckplatz 19 auf, in dem der EEG-Verstärker 13 eingesteckt werden kann. Auf diese Weise kann der Akkumulator geladen werden und/oder der EEG-Verstärker 13 über das Modul 18 mit Energie versorgt werden.

Es ist auch möglich, dass das Modul 18 ohne EEG-Verstärker ausgebildet ist und über seinen Dateneingang EEG-Signale von einem separaten externen EEG-Verstärker empfängt.

Ein derartiger modularer Aufbau ermöglicht die Verwendung bekannter Komponenten, wie konventionelle Überwachungsmonitore, mit einem Modul 18 zur Ableitung und Analyse von EEG-Signalen mittels symbolischer Transferentropie. Es ist auch möglich, dass das Modul 18 ausgebildet ist, um ausgewählte oder sämtliche Funktionen dieser Komponenten zu erfüllen.

Neben der Anwendung in einem EEG-Narkosemonitor 10 oder als Modul 18 in Verbindung mit einem Standardüberwachungsmonitor 16 kann die symbolische Transferentropie und der berechnete Indikator I und das Modul 18 auch in weiteren Anwendungsgebieten verwendet werden. Diese können insbesondere die Patientenüberwachung auf Intensivstationen, insbesondere bei Sedierung oder Koma, die Schlafüberwachung und die Vigilanzüberwachung von Verkehrsteilnehmern, beispielsweise Piloten oder Lkw- oder Busfahrern, umfassen.

Je nach Anwendungsgebiet kann das Modul 18 mit unterschiedlich ausgebildete Komponenten, wie zum Beispiel konventionellen Überwachungsmonitoren, verwendet werden.

Es ist insbesondere auch möglich, dass für das Modul 18 und für den EEG-Monitor lediglich ein Elektrodenpaar vorgesehen ist.

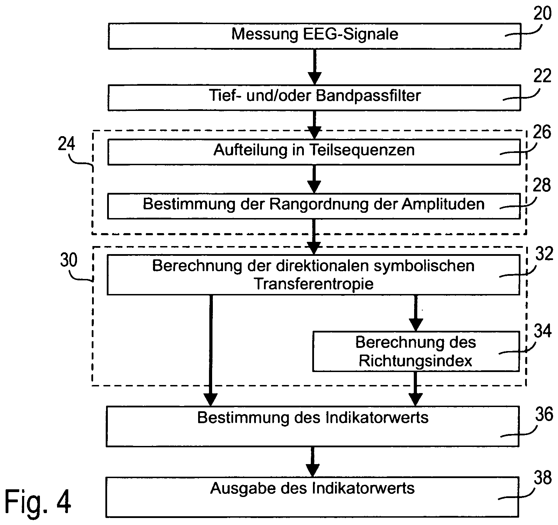

Ein Verfahren zur Bewusstseins- und/oder Schmerzüberwachung, insbesondere zur Narkoseüberwachung, in einem EEG-Narkosemonitor 10 oder in einem Modul 13 mit Standardüberwachungsmonitor 16 der Figuren 1 oder 2 wird im Folgenden anhand der Figuren 4, 5 und 6 beschrieben.

In einem ersten Schritt 20 werden die EEG-Signale gemessen. Dazu eignen sich vor allem intrafrontale (z.B. Fp1-Fp2 im international normierten 10-20 System), frontal-parietale (z.B. Fpz-Pz), frontal-temporale (z.B. Fp2-FT9), bitemporale (z.B. FT9-FT10) und frontal-okzipitale (z.B. Fpz-Oz) Elektrodenableitungen. Es werden vorzugsweise zwei dieser Paare benutzt.

In einem darauffolgenden Schritt 22 werden die EEG-Signale mit einer Grenzfrequenz von höchstens 30 Hz tiefpassgefiltert. Alternativ können die EEG-Signale bandpassgefiltert werden. Im Falle einer Bandpassfilterung eignen sich Frequenzen innerhalb des EEG $\alpha$- (8-13Hz) und/oder $\beta$-Bandes (13-30Hz) besonders. Auf diese Weise wird der Einfluss von Muskelaktivität im EEG, die besonders in hohen Frequenzen des EEG $\gamma$-Bandes oberhalb 30Hz zu einer schlechten SNR des EEG führt, reduziert.

Die von der symbolischen Transferentropie analysierten EEG-Signale sind zeitliche Messwertsequenzen von 2 bis 30 Sekunden Dauer, die mit einer vorbestimmten Abtastfrequenz $f_s$ bestimmt werden. In der beschriebenen Verfahrensvariante beträgt die zeitliche Dauer der Messwertsequenzen 10 Sekunden und die Abtastfrequenz $f_s$ entspricht 200 Hz. Die zeitliche Messwertsequenz umfasst somit 2000 Messpunkte.

Die Abtastfrequenz $f_s$ der EEG-Signale und die obere Filterfrequenz des Tiefpassfilters oder des Bandpassfilters sind so aufeinander abgestimmt, dass die Abtastfrequenz $f_s$ der EEG-Signale zumindest das Zweifache der oberen Filterfrequenz beträgt. Auf diese Weise wird ein Aliaseffekt vermieden.

Nach der Filterung der Messwertsequenzen erfolgt eine Symbolisierung 24. In der gezeigten Ausführungsvariante erfolgt eine Aufteilung 26 der zeitlichen Messwertsequenzen x, y eines Elektrodenpaares mit jeweils N Messwerten in Teilsequenzen x(i), y(i) der Länge m. Auf diese Weise werden jeweils bis zu N - m + 1 ($\tau$ = 1) Teilsequenzen x(i) bzw. y(i) erhalten, die im Falle eines Lags $\tau$ > 1 reduziert wird. Im vorliegenden Fall wird $\tau$ = 1 verwendet, wobei bei größeren Werten die aus den Teilsequenzen gebildeten Trajektorien im m-dimensionalen Euklidschen Raum entfaltet werden, wodurch unter Umständen eine genauere Analyse durch die symbolische Transferentropie erreicht wird. Werte von 1 bis 10 sind bei $f_s$ = 200 Hz besonders geeignet. Die Länge m der Teilsequenzen beträgt mindestens 3, sollte aber m! $\leq$ N für eine korrekte Berechnung einhalten, wobei in der beschriebenen Ausführungsform die Länge der Teilsequenzen gleich 3 ist. Auf diese Weise können gute Ergebnisse bei verhältnismäßig geringem Rechenaufwand erreicht werden.

Diese Teilsequenzen werden in einem darauffolgenden Verfahrensschritt 28 durch Bestimmung der Rangordnung der Amplituden symbolisiert (Ordinalanalyse), wodurch symbolische Sequenzen $\hat{x}(i)$ bzw. $\hat{y}(i)$ erhalten werden.

Die symbolischen Sequenzen $\hat{x}(i)$ und $\hat{y}(i)$ werden zu einer Berechnung 30 der symbolischen Transferentropie verwendet. Unter dem Begriff der symbolischen Transferentropie werden hierbei verschiedene Entropiemaße zusammengefasst.

In einem ersten Schritt 32 wird eine direktionale symbolische Transferentropie $STEn_{Y\to X}$ nach der Formel

$$STEn_{Y \to X} = \sum_i p(\hat{x}(i+\delta), \hat{x}_k(i), \hat{y}_l(i)) \cdot \log\left( \frac{p(\hat{x}(i+\delta)|\hat{x}_k(i), \hat{y}_l(i))}{p(\hat{x}(i+\delta)|\hat{x}_k(i))} \right)$$

berechnet. $\hat{x}_k(i)$ und $\hat{y}_l(i)$ entsprechen jeweils den k bzw. l letzten symbolischen Sequenzen entsprechend der Formeln

$$\hat{x}_k(i) = \hat{x}(i), \hat{x}(i-1), ..., \hat{x}(i-k); \hat{y}_l(i) = \hat{y}(i), \hat{y}(i-1), ..., \hat{y}(i-l)$$

In der vorliegenden Verfahrensvariante wird die Tiefe der Voraussagbarkeit auf eine im Abstand $\delta$ zu $\hat{x}(i+\delta)$ vorangehende Sequenz beschränkt, das heißt k und l werden gleich null gesetzt. Es ist jedoch auch möglich, dass k und l größer als null gewählt werden können.

Die direktionale symbolische Transferentropie wird aus der Shannon-Entropie und einer bedingten Kullbach-Leibler-Entropie hergeleitet.

Die gemeinsame Wahrscheinlichkeit, dass die symbolische Sequenz $\hat{x}(i+\delta)$ mit den vorhergehenden symbolischen Sequenzen $\hat{x}_k(i)$ und $\hat{y}_l(i)$ auftritt, ist $p(\hat{x}(i+\delta), \hat{x}_k(i), \hat{y}_l(i))$.

Die bedingte Wahrscheinlichkeit, dass die symbolische Sequenz $\hat{x}(i+\delta)$ unter der Bedingung der vorherge-

henden symbolischen Sequenzen $\hat{x}_k(i)$ und $\hat{y}_l(i)$ eintritt, ist $p(\hat{x}(i + \delta)|\hat{x}_k(i),\hat{y}_l(i))$.

Die bedingte Wahrscheinlichkeit, dass die symbolische Sequenz $\hat{x}(i + \delta)$ unter der Bedingung der vorhergehenden symbolischen Sequenz $\hat{x}_k(i)$ eintritt, ist durch $p(\hat{x}(i + \delta)|\hat{x}_k(i))$ gegeben.

Analog kann eine direktionale symbolische Transferentropie $STEn_{X \rightarrow Y}$ berechnet werden, wobei die entsprechenden Teilsequenzen der beiden Systeme X und Y vertauscht werden.

Ein Zeitversatz $\delta$ wird durch eine Anzahl von Messpunkten der zeitlichen Messwertsequenzen angegeben. Der tatsächliche zeitliche Versatz ergibt sich somit aus dem Zeitversatz $\delta$ und der Abtastfrequenz $f_s$.

In der hier vorgestellten Verfahrensvariante beträgt die Abtastfrequenz $f_s$ 200 Hz und der Zeitversatz $\delta$ entspricht 10 Messwerten. Auf diese Weise liegt der Quotient aus Abtastfrequenz $f_s$ und Zeitversatz $\delta$ mit 20 Hz im Frequenzbereich des EEG $\beta$-Bands. Unter Berücksichtigung der vorher genannten Bedingungen für die Abtastfrequenz können $\delta$ und $f_s$ im Wesentlichen beliebig variiert werden, solange deren Quotient im Frequenzbereich des analysierten EEG liegt, vorzugsweise im $\alpha$- oder $\beta$-Band.

Ein weiteres Maß der symbolischen Transferentropie stellt der Richtungsindex $STEn_{DI}$ dar, welcher in einem weiteren Verfahrensschritt 34 durch die Differenz der beiden zugeordneten direktionalen symbolischen Transferentropien berechnet wird:

$$STEn_{DI} = STEn_{X \rightarrow Y} - STEn_{Y \rightarrow X}$$

Der Richtungsindex $STEn_{DI}$ definiert und bestimmt die bevorzugte Richtung des Informationsflusses zwischen den beiden Systemen. Ein Wert von 0 repräsentiert bei vorhandener Kommunikation einen ausgeglichenen bidirektionalen Informationsaustausch zwischen X und Y. Für positive Werte ist das System X vorwiegend der Generator.

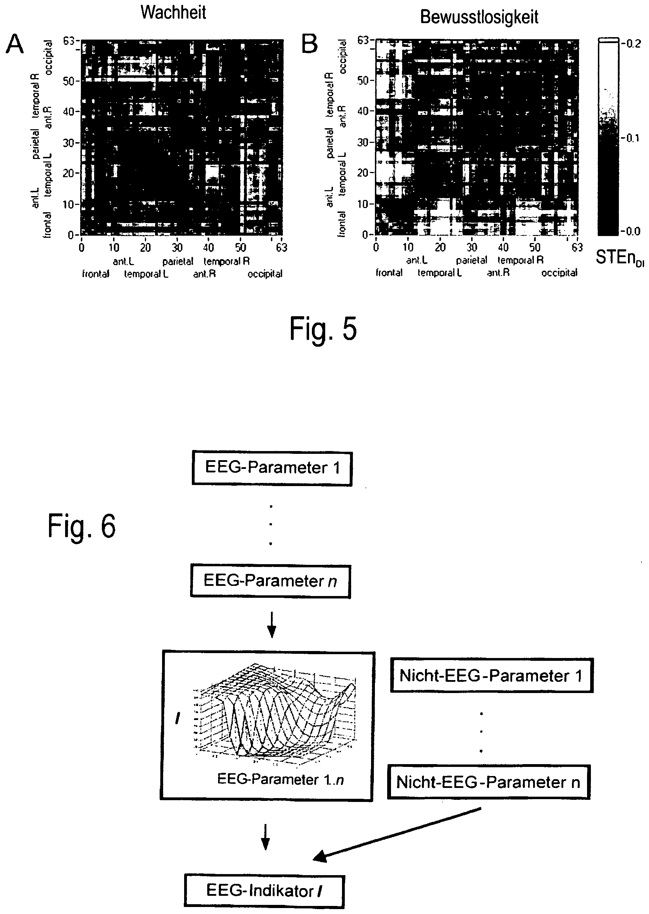

In Figur 5 wird eine grafische Darstellung des Richtungsindex $STEn_{DI}$ in Bildbereich A bei entspannter Wachheit und in Bildbereich B bei Bewusstlosigkeit dargestellt. Der Einfachheit halber wird der Betrag des Richtungsindex $STEn_{DI}$ aufgetragen, wobei niedrige Werte des Richtungsindex $STEn_{DI}$ dunkel und höhere Werte hell dargestellt sind. Die grafische Darstellung zeigt die Ergebnisse der symbolischen Transferentropie, die mit Hilfe von 64-Kanal EEG-Daten an 15 Probanden bei entspannter Wachheit und Propofol induzierter Bewusstlosigkeit berechnet wurde und Effekte von Propofol vor allem in Elektrodenkombinationen, die eine frontale Elektrode berücksichtigen.

Während bei entspannter Wachheit im Bildbereich A mehrheitlich ein ausgeglichener Informationsfluss zwischen den Elektrodenpaaren beobachtet wird mit entsprechenden niedrigen Werten des Richtungsindex $STEn_{DI}$, dominiert während Bewusstlosigkeit in Bild B ein unbalancierter Austausch von Informationen, gekennzeichnet durch die hellere Färbung und entsprechend höhere Werte des Richtungsindex $STEn_{DI}$. Dies wird insbesondere bei frontaltemporalen, frontal-parietalen und okzipitalen Elektrodenkombinationen beobachtet. Dieses Ergebnis stimmt qualitativ mit bildgebenden und räumlich hochauflösenden fMRT-Untersuchungen während Anästhesie überein, die auf eine Unterdrückung der kortiko-kortikalen Konnektivität der Netzwerkarchitektur insbesondere des Default und höherer exekutiver Netzwerke hinweisen.

An die Berechnung 30 der symbolischen Transferentropie anschließend erfolgt die Bestimmung 36 eines Indikatorwerts I, wie in Figur 6 dargestellt. Hierbei wird eine Mehrzahl von EEG-Parametern und/oder Nicht-EEG-Parametern der Standardüberwachung (Herzkreislaufsystem, Atmung sowie Patientendaten und Medikamenteninformationen), 1 bis n ausgewertet und ein einzelner Indikatorwert I bestimmt. Die Parameter 1 bis n umfassen insbesondere die in den vorhergehenden Verfahrensschritten 32, 34 bestimmten symbolischen Transferentropiemaße $STEn_{DI}$, $STEn_{X \rightarrow Y}$ und $STEn_{Y \rightarrow X}$.

Der Indikatorwert I ist beispielsweise so definiert, dass er Werte zwischen 0 und 100 annehmen kann, wobei Werte zwischen 80 und 100 Wachheit und Werte zwischen 0 und 20 einer tiefen Narkose entsprechen. Neben den oben genannten Parametern der symbolischen Transferentropie können auch weitere EEG-Parameter oder weitere Nicht-EEG-Parameter (Standardüberwachungsparameter inkl. Patientendaten und Medikamenteninformationen) zur Bestimmung des Indikatorwerts I beitragen.

In einem abschließenden Verfahrensschritt 38 wird der Indikatorwert I ausgegeben, wobei der Indikatorwert entweder als eigenständiger Ausgabewert angezeigt wird oder in einem Narkosemonitor zusammen mit anderen Parametern in die Bestimmung eines weiteren Indikatorwerts eingeht.

Das oben beschriebene Verfahren zur Bewusstseinsüberwachung ist für beide Geschlechter und alle Altersklassen geeignet. Es ist jedoch möglich, dass je nach Geschlecht oder Alter bzw. entsprechend dem Anwendungsbereich unterschiedliche Parameter angewandt werden, wobei die Parameter von der Anordnung und Anzahl der Elektroden bis hin zu den Parametern bei der Berechnung der direktionalen symbolischen Transferentropie beispielsweise des Zeitversatzes $\delta$ variiert werden können. Das Verfahren lässt sich zudem bei unterschiedlichen Kombinationen hypnotisch und analgetisch wirkender Anästhetika einsetzen und speziell für spezifische Medikamentenprotokolle konfigurieren.

Der Ansatz der symbolischen Transferentropie liegt nahe an den zugrunde liegenden neuronalen Prozessen.

Dazu lässt sich die kortiko-kortikale Kopplung auf informatorischer Ebene durch Zeitreihen der elektrischen Potenziale spezifischer Elektroden erfassen und quantifizieren. Die symbolische Transferentropie adressiert dabei gezielt mechanistische Effekte von medikamentös induziertem Bewusstseinsverlust. Dieser Ansatz ist vorteilhaft, weil Bewusstlosigkeit mit beeinträchtigter Informationsverarbeitung direkt zusammenhängt. Die aus den hochauflösenden EEG-Daten durchgeführten Voruntersuchungen an Probanden unter Propofolnarkose zeigen, dass die symbolische Transferentropie als neuer EEG-Parameter für die Narkoseüberwachung eine besonders gute, den heutigen Stand der Technik übertreffende Unterscheidung zwischen Wachheit und Bewusstlosigkeit erreicht.

Die symbolische Transferentropie lässt sich ebenso für angrenzende Anwendungen im Zusammenhang mit Sedierungs-, Schlaf- und Komaüberwachung einsetzen.

**Patentansprüche**

1. Verfahren zur Bewusstseins- und/oder Schmerzüberwachung, vorzugsweise zur Narkoseüberwachung, bei dem EEG-Signale mittels symbolischer Transferentropie ausgewertet werden, um zwischen Wachheit und Bewusstlosigkeit, zu unterscheiden, wobei ein Indikatorwert zur Unterscheidung zwischen Wachheit und Bewusstlosigkeit basierend auf der Auswertung der EEG-Signale mittels symbolischer Transferentropie ermittelt wird.

2. Verfahren nach Anspruch 1, **dadurch gekennzeichnet, dass** die EEG-Signale von einer Mehrzahl von Elektroden, vorzugsweise einem oder einer Mehrzahl von Elektrodenpaaren, ausgewertet werden.

3. Verfahren nach Anspruch 1 oder 2, **dadurch gekennzeichnet, dass** intrafrontale, frontal-parietale, frontal-temporale, bitemporale oder frontal-okzipitale Elektrodenableitungen vorgesehen sind.

4. Verfahren nach einem der vorhergehenden Ansprüche, **dadurch gekennzeichnet, dass** die EEG-Signale vor einer Berechnung der symbolischen Transferentropie mit einer Grenzfrequenz von höchstens 30 Hz tiefpassgefiltert und/oder bandpassgefiltert werden, wobei die Bandbreite vorzugsweise innerhalb des Frequenzbandes von 8Hz bis 30Hz liegt.

5. Verfahren nach Anspruch 4, **dadurch gekennzeichnet, dass** eine Abtastfrequenz der EEG-Signale vorgesehen ist, welche zumindest das Zweifache, vorzugsweise zumindest das Fünffache, der oberen Filterfrequenz beträgt.

6. Verfahren nach einem der vorhergehenden Ansprüche, **dadurch gekennzeichnet, dass** die EEG-Signale zeitliche Messwertsequenzen von 2 bis 30 Sekunden Dauer sind.

7. Verfahren nach einem der vorhergehenden Ansprüche, **dadurch gekennzeichnet, dass** aus zeitlichen Messwertsequenzen x, y mit N Messwerten der EEG-Signale Teilsequenzen x(i), y(i) der Länge m mit Lag $\tau$ entlang x und y gebildet werden und symbolische Sequenzen $\hat{x}(i)$, $\hat{y}(i)$ durch eine Symbolisierung der Teilsequenzen x(i), y(i) vorzugsweise durch Bestimmung der Rangordnung der Amplituden, erhalten werden, wobei m größer oder gleich 3, vorzugsweise genau 3, ist.

8. Verfahren nach einem der vorhergehenden Ansprüche, **dadurch gekennzeichnet, dass** eine direktionale symbolische Transferentropie nach der Formel

$$\text{STEn}_{Y \to X} = \sum_i p(\hat{x}(i+\delta), \hat{x}_k(i), \hat{y}_l(i)) \cdot \log\left( \frac{p(\hat{x}(i+\delta)|\hat{x}_k(i), \hat{y}_l(i))}{p(\hat{x}(i+\delta)|\hat{x}_k(i))} \right)$$

berechnet wird, wobei k und l für

$$\hat{x}_k(i) = \hat{x}(i), \hat{x}(i-1),...,\hat{x}(i-k); \hat{y}_l(i) = \hat{y}(i), \hat{y}(i-1),...,\hat{y}(i-l)$$

vorzugsweise null sind.

9. Verfahren nach Anspruch 8, **dadurch gekennzeichnet, dass** der Zeitversatz ö so gewählt ist, dass der Quotient aus Abtastfrequenz und Zeitversatz im Frequenzbereich des EEG $\alpha$- oder $\beta$-Bandes liegt..

10. Verfahren nach Anspruch 8 oder 9, **dadurch gekennzeichnet, dass** ein Richtungsindex $STEn_{DI}$ nach der Formel $STEn_{DI} = STEn_{X \rightarrow Y} - STEn_{Y \rightarrow X}$ berechnet wird.

11. Modul (18) zur Analyse von EEG-Signalen mit einem Dateneingang, welcher EEG-Signale empfangen kann, einer Rechnereinheit, welche geeignet ist die EEG-Signale nach einem Verfahren gemäß einem der vorhergehenden Ansprüche auszuwerten und einer Ausgabeeinheit, welche geeignet ist den Indikatorwert zur Unterscheidung zwischen Wachheit und Bewusstlosigkeit auszugeben.

12. Modul (18) nach Anspruch 11, **dadurch gekennzeichnet, dass** ein EEG-Verstärker (13) vorgesehen ist, welcher im Modul (18) fest integriert ist oder eine separate portable vorzugsweise drahtlose Einheit bildet und EEG-Signale an den Dateneingang des Moduls (18) bereitstellt.

13. Modul (18) nach Anspruch 11 oder 12, **dadurch gekennzeichnet, dass** das Modul (18) eine Schnittstelle für einen konventionellen Überwachungsmonitor (16) aufweist, die nicht-EEG-Parameter empfangen kann, wobei die Rechnereinheit ausgebildet ist, den Indikatorwert unter Einbezug der nicht-EEG-Parameter zu bestimmen.

14. EEG-Narkosemonitor (10), welcher ausgebildet ist, um EEG-Signale zu messen und mittels symbolischer Transferentropie nach einem Verfahren gemäß der Ansprüche 1 bis 10 auszuwerten, um zwischen Wachheit und Bewusstlosigkeit zu unterscheiden, wobei der EEG-Narkosemonitor (10) ausgebildet ist, um den Indikatorwert, zur Unterscheidung zwischen Wachheit und Bewusstlosigkeit, zu ermitteln.

**Claims**

1. A method for consciousness and/or pain monitoring, preferably for anesthesia monitoring, in which EEG signals are evaluated by means of symbolic transfer entropy, to differentiate between awareness and unconsciousness, an indicator value being established for differentiating between awareness and unconsciousness based on the evaluation of the EEG signals by means of symbolic transfer entropy.

2. The method according to claim 1, **characterized in that** the EEG signals from a plurality of electrodes, preferably one or a plurality of electrode pairs, are evaluated.

3. The method according to claim 1 or 2, **characterized in that** intrafrontal, frontal-parietal, frontal-temporal, bitemporal or frontal-occipital electrode leads are provided.

4. The method according to any of the preceding claims, **characterized in that** prior to a calculation of the symbolic transfer entropy, the EEG signals are low-pass filtered and/or band-pass filtered with a cutoff frequency of 30 Hz at maximum, the bandwidth preferably being within the frequency band of 8 Hz to 30 Hz.

5. The method according to claim 4, **characterized in that** a sampling frequency of the EEG signals is provided which amounts to at least twice, preferably at least five times the upper filter frequency.

6. The method according to any of the preceding claims, **characterized in that** the EEG signals are temporal measured value sequences of a duration of 2 to 30 seconds.

7. The method according to any of the preceding claims, **characterized in that** from temporal measured value sequences x, y with N measured values of the EEG signals, subsequences x(i), y(i) of a length m with lag $\tau$ are formed along x and y and symbolic sequences $\hat{x}(i)$, $\hat{y}(i)$ are obtained by a symbolization of the subsequences x(i), y(i), preferably by determining the rank order of the amplitudes, where m is equal to or greater than 3, preferably exactly 3.

8. The method according to any of the preceding claims, **characterized in that** a directional symbolic transfer entropy is calculated according to the formula

$$\mathrm{STEn}_{Y \to X} = \sum_i p(\hat{x}(i+\delta), \hat{x}_k(i), \hat{y}_l(i)) \cdot \log\left( \frac{p(\hat{x}(i+\delta) | \hat{x}_k(i), \hat{y}_l(i))}{p(\hat{x}(i+\delta) | \hat{x}_k(i))} \right)$$

where k and l for

$$\hat{x}_k(i) = \hat{x}(i), \hat{x}(i-1), ..., \hat{x}(i-k); \hat{y}_l(i) = \hat{y}(i), \hat{y}(i-1), ..., \hat{y}(i-l)$$

are preferably zero.

9. The method according to claim 8, **characterized in that** the time offset $\delta$ is selected such that the quotient of sampling frequency and time offset is within the frequency range of the EEG $\alpha$- or $\beta$-band.

10. The method according to claim 8 or 9, **characterized in that** a direction index $\mathrm{STEn}_{DI}$ is calculated according to the formula $\mathrm{STEn}_{DI} = \mathrm{STEn}_{X \to Y} - \mathrm{STEn}_{Y \to X}$.

11. A module (18) for analyzing EEG signals, comprising a data input that can receive EEG signals, a computer unit that is suitable for evaluating the EEG signals in accordance with a method according to any of the preceding claims, and an output unit that is suitable for outputting the indicator value for differentiating between awareness and unconsciousness.

12. The module (18) according to claim 11, **characterized in that** an EEG amplifier (13) is provided which is firmly integrated in the module (18) or forms a separate, portable, preferably wireless unit and provides EEG signals to the data input of the module (18).

13. The module (18) according to claim 11 or 12, **characterized in that** the module (18) includes an interface for a conventional patient monitor (16), the interface being adapted to receive non-EEG parameters, the computer unit being configured to determine the indicator value taking into account the non-EEG parameters.

14. An EEG anesthesia monitor (10) which is configured to measure EEG signals and to evaluate them by means of symbolic transfer entropy in accordance with a method according to claims 1 to 10, for differentiating between awareness and unconsciousness, the EEG anesthesia monitor (10) being configured to establish the indicator value for differentiating between awareness and unconsciousness.

**Revendications**

1. Procédé de surveillance de la conscience et/ou de la douleur, de préférence pour la surveillance d'une anesthésie, dans lequel des signaux EEG sont évalués par entropie de transfert symbolique pour différencier entre un état de veille et l'inconscience, une valeur indicatrice pour la distinction entre l'état de veille et l'inconscience étant déterminée sur la base de l'évaluation des signaux EEG par entropie de transfert symbolique.

2. Procédé selon la revendication 1, **caractérisé en ce que** les signaux EEG sont évalués par une pluralité d'électrodes, de préférence par une ou plusieurs paires d'électrodes.

3. Procédé selon la revendication 1 ou 2, **caractérisé en ce qu'**il est prévu des dérivations d'électrode intra-frontales, fronto-pariétales, fronto-temporales, bitemporales ou fronto-occipitales.

4. Procédé selon l'une des revendications précédentes, **caractérisé en ce que** les signaux EEG sont filtrés par un filtre passe-bas et/ou sont filtrés par un filtre passe-bande avec une fréquence limite de 30 Hz au maximum avant un calcul de l'entropie de transfert symbolique, la largeur de bande se trouvant de préférence dans la bande de fréquence de 8 Hz à 30 Hz.

5. Procédé selon la revendication 4, **caractérisé en ce qu'**il est prévu une fréquence de balayage des signaux EEG laquelle correspond au moins au double, de préférence au moins au quintuple de la fréquence de filtrage supérieure.

**6.** Procédé selon l'une des revendications précédentes, **caractérisé en ce que** les signaux EEG sont des séquences de valeurs mesurées temporelles d'une durée de 2 à 30 secondes.

**7.** Procédé selon l'une des revendications précédentes, **caractérisé en ce qu'**à partir de séquences de valeurs mesurées temporelles x, y avec N valeurs mesurées des signaux EEG, des séquences partielles x(i), y(i) de longueur m avec un lag de $\tau$ sont formées le long de x et de y et des séquences symboliques $\hat{x}(i)$, $\hat{y}(i)$ sont obtenues par une symbolisation des séquences partielles x(i), y(i), de préférence par la détermination de l'ordre hiérarchique des amplitudes, m étant supérieur ou égal à 3, de préférence exactement 3.

**8.** Procédé selon l'une des revendications précédentes, **caractérisé en ce qu'**une entropie de transfert symbolique directionnelle est calculée selon la formule

$$\text{STEn}_{Y \to X} = \sum_i p(\hat{x}(i+\delta), \hat{x}_k(i), \hat{y}_l(i)) \cdot \log\left( \frac{p(\hat{x}(i+\delta)|\hat{x}_k(i), \hat{y}_l(i))}{p(\hat{x}(i+\delta)|\hat{x}_k(i))} \right)$$

k et l pour

$$\hat{x}_k(i) = \hat{x}(i), \hat{x}(i-1), ..., \hat{x}(i-k); \hat{y}_l(i) = \hat{y}(i), \hat{y}(i-1), ..., \hat{y}(i-l)$$

étant de préférence nuls.

**9.** Procédé selon la revendication 8, **caractérisé en ce que** le décalage dans le temps $\delta$ est choisi de sorte que le quotient de la fréquence de balayage et du décalage dans le temps est dans la gamme des fréquences de la bande EEG $\alpha$ ou $\beta$.

**10.** Procédé selon la revendication 8 ou 9, **caractérisé en ce qu'**un indice de direction $\text{STEn}_{DI}$ est calculé selon la formule $\text{STEn}_{DI} = \text{STEn}_{X \to Y} - \text{STEn}_{Y \to X}$.

**11.** Module (18) pour l'analyse de signaux EEG, comportant une entrée de données qui peut recevoir des signaux EEG, une unité informatique qui est apte à évaluer les signaux EEG conformément à un procédé selon l'une des revendications précédentes, et une unité de sortie qui est apte à sortir la valeur indicatrice pour la distinction entre l'état de veille et l'inconscience.

**12.** Module (18) selon la revendication 11, **caractérisé en ce qu'**il est prévu un amplificateur EEG (13) qui est intégré de manière fixe au module (18) ou forme une unité séparée portable de préférence sans fil et fournit des signaux EEG à l'entrée de données du module (18).

**13.** Module (18) selon la revendication 11 ou 12, **caractérisé en ce que** le module (18) présente une interface pour un moniteur de surveillance (16) classique qui est apte à recevoir des paramètres non-EEG, l'unité informatique étant réalisée de manière à déterminer la valeur indicatrice en tenant compte des paramètres non-EEG.

**14.** Moniteur d'anesthésie EEG (10) lequel est réalisé pour mesurer des signaux EEG et pour les évaluer par entropie de transfert symbolique conformément à un procédé selon les revendications 1 à 10 afin de faire une distinction entre un état de veille et l'inconscience, le moniteur d'anesthésie EEG (10) étant réalisé pour déterminer la valeur indicatrice pour la distinction entre un état de veille et l'inconscience.

**Fig. 1**

**Fig. 2**

**Fig. 3**

Fig. 4

| | 20 |
| Messung EEG-Signale | |

| | 22 |
| Tief- und/oder Bandpassfilter | |

24

| | 26 |
| Aufteilung in Teilsequenzen | |

| | 28 |
| Bestimmung der Rangordnung der Amplituden | |

30

| | 32 |
| Berechnung der direktionalen symbolischen Transferentropie | |

| | 34 |
| Berechnung des Richtungsindex | |

| | 36 |
| Bestimmung des Indikatorwerts | |

| | 38 |
| Ausgabe des Indikatorwerts | |

Wachheit            Bewusstlosigkeit

# Fig. 5

# Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009079384 A1

**In der Beschreibung aufgeführte Nicht-Patentliteratur**

- Symbolische Transferentropie: Charakterisierung gerichteter Interaktionen in nichtlinearen dynamischen Netzwerken. *Dissertation,* 14. April 2010